# EUROPÄISCHE PATENTANMELDUNG

(11) **EP 0 803 568 A1**
(43) Veröffentlichungstag der Anmeldung: **29.10.1997**
(21) Anmeldenummer: 96810267.3
(22) Anmeldetag: 26.04.1996
(51) Int. Cl.: C12M 1/107

(54) **Vergärungsanlage und ein mit dieser Anlage durchführbares mehrstufiges Verfahren**

(71) Anmelder: CT Umwelttechnik AG, CH-8404 Winterthur (CH)
(72) Erfinder: Caviezel, Mario, 8425 Oberembrach (CH); Wetter, Rolf, 8357 Guntershausen (CH); Nünlist, Bruno, 8546 Kefikon (CH)
(74) Vertreter: Heubeck, Bernhard

(57) **Zusammenfassung**

Die Vergärungsanlage (1) zum Vergären von organischen Abfällen umfasst eine Beschickungsvorrichtung (3), diverse Reaktoren (200, 201, 202, 203), mindestens einen Speicherbehälter (209), eine Entwässerungseinrichtung (7) und Fördermittel (45, 52, 210, 20a, 20b) als Anlagekomponenten. Die Beschickungsvorrichtung besteht aus einer stationären Ladeeinrichtung (4) und einem an die Ladeeinrichtung ankoppelbaren Transportbehälter (30, 30') für die Abfälle. Die Reaktoren sind für eine Hydrolyse sowie auf diese folgende Vergärungsstufen vorgesehen. In der Entwässerungseinrichtung ist ein bei der Vergärung als Rest bleibender Bioschlamm eindickbar. Die Fördermittel stellen Verbindungen zwischen den Anlagekomponenten her. Erfindungsgemäss ist jeder als Reaktor oder Speicher dienende Behälter über eine Förderleitung (210) mit einem gemeinsamen Transportkanal (20a) verbunden. Am Austrittsende des Transportkanals ist eine zwangsfördernde Pumpe (20b) angeordnet, mittels der zu behandelndes Material aus dem Transportkanal gezielt in jeden der genannten Behälter oder in die Entwässerungseinrichtung einspeisbar ist.

## Beschreibung

Die Erfindung betrifft eine Vergärungsanlage gemäss dem Oberbegriff von Anspruch 1 sowie ein mit dieser Anlage durchgeführbares mehrstufiges Verfahren.

Es ist beispielsweise aus der CH-PS 662 339 (= P.5917) eine anaerobe Behandlung von hochbelasteten Abwässern bekannte, bei der die organischen Inhaltsstoffe durch Vergärung teilweise in Biogas umgewandelt werden. Dieses Verfahren kann in ähnlicher Weise verwendet werden für die Behandlung von organischen Abfällen, die in der Landwirtschaft (z.B. pflanzliche Abfälle oder Gemische mit Tierexkrementen) oder in Gärten ("Grüngut") anfallen. Da sich das Verfahren in einer Vergärungsanlage, die gegen die Umgebung abgeschlossen ist, durchführen lässt, ist eine Geruchsbelästigung vermeidbar. Es sind dabei allerdings verschiedene Massnahmen zu treffen, beispielsweise die Behandlung von Abgasen in Biofiltern, in denen organische Stoffe, die Ursache für Geruchsbelästigungen sind, durch Mikroorganismen abgebaut werden.

In der europäischen Patentanmeldung Nr. 96810208.7 (= P.6727) ist eine Vorrichtung zum Beschicken einer Vergärungsanlage beschrieben, welche eine stationäre Ladeeinrichtung, die Teil der Anlage ist, und einen transportierbaren, an die Ladeeinrichtung ankoppelbaren Behälter umfasst. Mit dem Transportbehälter werden die bei einem Erzeuger gesammelten Abfälle zu der Anlage transportiert und ihr zugeführt. Die Ladeeinrichtung enthält mechanische Mittel, beispielsweise mit Motoren antreibbare Förderschrauben, mit welchen sich die Abfälle steuerbar dosiert in die Anlage weiterfördern lassen. Der Transportbehälter weist separat angeordnete, verschliessbare Öffnungen für das Füllen bzw. Entleeren auf. Nach dem Ankoppelung des Transportbehälters an die Ladeeinrichtung ist die Anlage weitgehend geruchsdicht gegen die Umgebung abgeschlossen.

In Abhängigkeit von der Zusammensetzung der zu behandelnden Abfällen sind für die Einstellung einzelner Verfahrensparameter besondere Massnahmen zu treffen. So kann es nötig sein, einem Reaktor der Anlage Material zu entnehmen und gezielt einem anderen zuzuführen, wobei die Zuordnung der beiden Reaktoren in der Regel von Fall zu Fall verschieden ist. Es ist daher eine grosse Flexibilität hinsichtlich den Möglichkeiten, die Anlagenkomponenten miteinander zu verbinden, erforderlich. Aufgabe der Erfindung ist es, eine Anlage zu schaffen, die sich durch eine solche Flexibilität auszeichnet, wobei die Herstellung der Verbindungen möglich sein muss, ohne dass dabei eine vorübergehende Öffnung der Anlage gegen die Umgebung nötig ist. Diese Aufgabe wird durch die im kennzeichnenden Teil des Anspruchs 1 genannten Massnahmen gelöst.

Die Vergärungsanlage zum Vergären von organischen Abfällen umfasst eine Beschickungsvorrichtung, diverse Reaktoren, mindestens einen Speicherbehälter, eine Entwässerungseinrichtung und Fördermittel als Anlagekomponenten. Die Beschickungsvorrichtung besteht aus einer stationären Ladeeinrichtung und einem an die Ladeeinrichtung ankoppelbaren Transportbehälter für die Abfälle. Die Reaktoren sind für eine Hydrolyse sowie auf diese folgende Vergärungsstufen vorgesehen. In der Entwässerungseinrichtung ist ein bei der Vergärung als Rest bleibender Bioschlamm eindickbar. Die Fördermittel stellen Verbindungen zwischen den Anlagekomponenten her. Erfindungsgemäss ist jeder als Reaktor oder Speicher dienende Behälter über eine Förderleitung mit einem gemeinsamen Transportkanal verbunden. Am Austrittsende des Transportkanals ist eine zwangsfördernde Pumpe angeordnet, mittels der zu behandelndes Material aus dem Transportkanal gezielt in jeden der genannten Behälter oder in die Entwässerungseinrichtung einspeisbar ist.

Die abhängigen Ansprüche 2 bis 4 beziehen sich auf vorteilhafte Ausführungsformen der erfindungsgemässen Anlage. Gegenstand der Ansprüche 5 bis 10 ist ein Verfahren, das mit der Anlage gemäss den Ansprüchen 1 bis 4 durchführbar ist.

Nachfolgend wird die Erfindung anhand der Zeichnungen erläutert. Es zeigen:
- Fig. 1: eine Schema der erfindungsgemässen Anlage und
- Fig. 2: detaillierter einen Teil der Anlage, der in Fig.2 als Block angegeben ist.

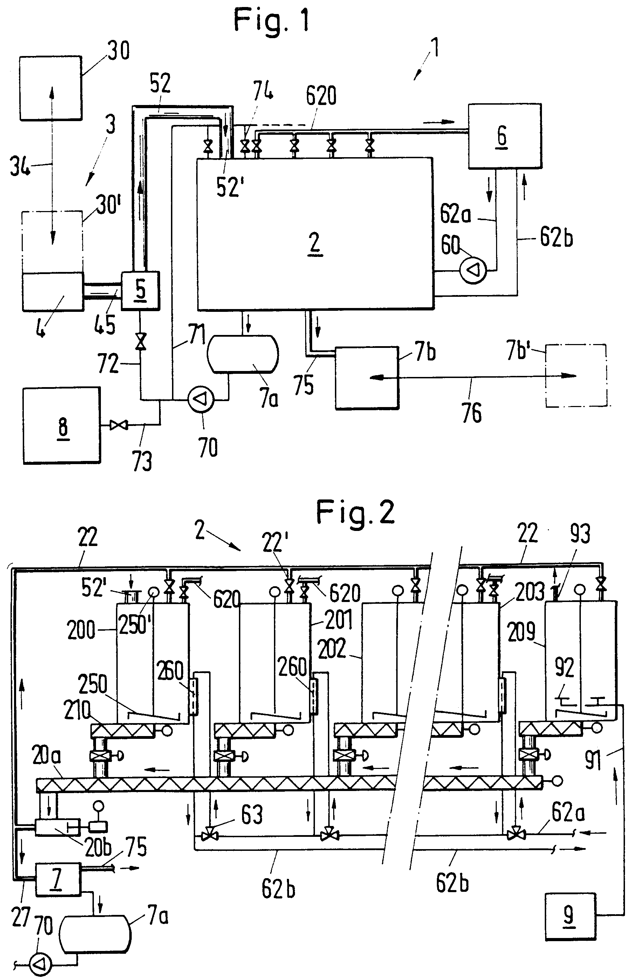

Fig.1 stellt die gesamte Vergärungsanlage 1 gemäss der Erfindung dar, die eine Teilanlage 2 mit Reaktoren 200, bis 203 - siehe Fig. 2 - umfasst. Der Anlage 1 werden mittels einer Beschickungsvorrichtung 3 organisches Rohmaterial, nämlich die zu behandelnden Abfälle, zugeführt. Diese Vorrichtung 3 besteht aus einer stationären Ladeeinrichtung 4 und aus an die Ladeeinrichtung 4 ankoppelbaren Transportbehältern 30 (angekoppelt: 30'), mit denen sich dezentral bei Erzeugern gesammelte Abfälle zu der Anlage 1 transportieren lassen. Der Transport der Behälter 30, 30' ist durch den Doppelpfeil 34 angedeutet.

Aus der Ladeeinrichtung 4 gelangt das organische Rohmaterial über eine Förderleitung 45 in einen Zerkleinerer 5 - beispielsweise eine Schlagmühle. Aus einem Vorratsbehälter 7a wird mittels einer Pumpe 70 und durch die Leitung 72 Wasser in den Zerkleinerer 5 eingeleitet. Dort wird das Abfallmaterial mit dem Wasser versetzt, so dass der Feststoffanteil anschliessend maximal rund 25% TS (Trockensubstanz) beträgt. Es kann nach dem Zerkleinerer 5 auch eine Siebung vorgesehen sein (nicht dargestellt). Allfällige Siebrückstände werden aus der Anlage 1 entfernt und mit Vorteil einer Kompostierung zugeführt.

Über die Förderleitung wird mit Schraubenförderern (Schneckenförderern) das Abfallmaterial in die Teilanlage 2 transportiert, wo es durch den Stutzen 52' in einen Versäuerungsbehälter 200 eintritt. In diesem Reaktor 200 findet unter der Einwirkung von Mikroorganismen eine Hydrolyse bei 36-38°C statt. Dieser sogenannte "mesophile Prozess" verläuft exotherm. Eine Hydrolyse ist auch bei 50-55°C möglich.

Durch die Versäuerungsreaktionen der Hydrolysestufe werden langkettige Kohlenstoffverbindungen wie beispielsweise Stärke oder Cellulose anaerob in Fettsäuren (Essig-, Propion-, Buttersäure, ...) umgewandelt; diese Zwischenprodukte sind sehr geruchsintensiv. Weitere Hydrolyseprodukte sind Wasserstoff, Kohlendioxid, Alkohole, Aminosäuren und weitere organische Stoffe.

Das durch die Abfälle gegebene organische Rohmaterial kann beim Einspeisen in die Anlage 1 durch Fermentation bereits vorgewärmt sein. Eine weitere Erhöhung der Temperatur auf die für die Hydrolyse benötigte Prozesstemperatur wird durch Wärmetauscherelemente 260, die aussen am Behälter 200 angebrachten sind, hergestellt. Die Temperatur wird überwacht und geregelt.

Das entstandene Hydrolysat bildet ein geeignetes Substrat für die nachfolgenden thermophilen Vergärungsstufen in den Reaktoren 201 bis 203. Über eine Förderleitung 210 sowie einen Transportkanal 20a, die jeweils einen Schraubenförderer enthalten, und mittels einer zwangsfördernden Pumpe 20b (Kolbenpumpe) wird das Hydrolysat über Leitungen 22 und 22' in die erste dieser Stufen - Reaktor 201 - transportiert. Der Reaktor 201 wird auf die Temperatur von beispielsweise 60°C gebracht. Dabei wird die benötigte Wärme über einen Wärmetauscher 260 wie beim Versäuerungsbehälter 200 zugeführt.

Erfindungsgemäss ist jeder als Reaktor oder Speicher dienende Behälter der Teilanlage 2 über jeweils eine Förderleitung 210 mit dem gemeinsamen Transportkanal 20a verbunden ist. Am Austrittsende des Transportkanals 20a ist die zwangsfördernde Pumpe 20b angeordnet, über die das zu behandelnde Material aus dem Transportkanal 20a gezielt in jeden der genannten Behälter, nämlich die Reaktoren 200 bis 203 sowie einen Speicher 209, oder in eine Entwässerungseinrichtung 7 (Leitung 27) einspeisbar ist.

Alle Reaktorbehälter 200 bis 203 und Speicherbehälter 209 enthalten Rührwerke 250 (Antriebe 250'), mit denen nach einer teilweisen Entleerung eines Behälters zugeführtes Material in den verbliebenen Behälterinhalt eingemischt werden kann.

In den Reaktoren 201 bis 203 durchläuft das zu behandelnde Material die thermophilen Stufen. Diese bilden ein mehrstufiges Vergärungsverfahren (schwach exotherme, anaerobe Prozesse), bei Temperaturen von 55 - 70°C. Die Fettsäuren des bei der Hydrolyse gebildeten Substrats werden durch Vergären zu Biogas "methanisiert", d.h. durch Mikroorganismen (Biomasse) in ein hauptsächlich aus Methan und Kohlensäure bestehendes Gemisch (Biogas) umgewandelt. Das Substrat wird von Stufe zu Stufe fliessfähiger, da durch die Umsetzung in Biogas die festen Bestandteile teilweise eliminiert werden. Durch den Abbau der Fettsäuren reduziert sich der Geruch. Der als Endprodukt entstehende Bioschlamm, der "ausgefaulte Schlamm", erscheint in einer stabilisierten Form, in der die Vergärung abgeschlossen ist.

Der Energieverlust der Reaktoren 201 bis 203 durch Abstrahlung ist grösser als die Wärmeerzeugung der Prozesse. Damit die notwendige Prozesstemperatur gehalten werden kann, muss der Wärmeverluste mit einer Wärmezufuhr (Wärmetauscher 260) kompensiert werden. Es ist bei allen Vergärungsstufen eine Temperaturregelung vorgesehen.

Als Energiequelle für die thermophilen Verfahrensschritte wie auch für die vorgeschaltete Hydrolyse werden mit Vorteil die im Verfahren gebildeten Gase, insbesondere das Biogas verwendet. Das gebildete Biogas wird aus den Reaktoren 200 bis 203 über Leitungen 620 in eine Heizanlage 6 eingeleitet, in der durch Verbrennen des Gases oder eines Teils des Gases Heisswasser erzeugt wird. (Ein Überschuss an Biogas kann anderen Verwendungszwecken zugeführt werden.) Das Heisswasser wird über eine Verteilleitung 62a, mittels einer Umwälzumpe 60, unter Verwendung von Dreiwegeventilen 63 gesteuert durch die einzelnen Wärmetauscher 260 der Reaktoren 200 bis 203 und anschliessend über die Leitung 62b zurück in die Heizanlage 6 transportiert.

Die Menge des gebildeten Biogases (rund 2/3 Methan, 1/3 Kohlendioxid) hängt von dem Ausfaulgrad, der Bakterienmenge, der Retentionszeit und von weiteren Parametern ab.

Die methanerzeugende Biomasse besteht aus langsam wachsenden Mikroorganismen: Pro 1 kg abgebautem Kohlenstoff werden im Mittel rund 35 g dieser methanogenen Bakterien gebildet. Auf das organische Rohmaterial umgerechnet macht das rund 1% aus. Dabei ist vorausgesetzt, dass die Hälfte der kohlenstoffhaltigen Inhaltsstoffe vergärbar sind.

In der Regel kann angenommen werden, dass bei einem Ausgangsprodukt des Verfahrens mit 40% TS rund 1/5 als vergärbare Masse (OTS) vorliegt. Aus dieser Masse wird pro kg rund 0,3 m³ Biogas (bei Normalbedingungen) gebildet.

Der Prozess muss so gesteuert werden, dass genügend aktive Biomasse (methanogene Bakterien) für das Vergärungsverfahren zur Verfügung steht. Damit durch die Entnahme von Schlamm aus den Reaktoren 201 bis 203 nicht zu viele der Bakterien für das Vergärungsverfahren verloren gehen, wird das bei der Schlammentwässerung 7 abgetrennte Wasser, das Biomasse enthält, wieder in die thermophilen Stufen rückgeführt. Dazu ist ein besonderer Wasserkreislauf der Anlage 1 vorgesehen, der den Wasserspeicher 7a, die Pumpe 70, die Leitung 72 und Leitungen 71 sowie 74 umfasst.

Wasser, das bei der Schlammentwässerung 7 im Überschuss entsteht, wird über eine Leitung 73 einer Abwasserbehandlung 8 zugeführt. Der eingedickte Schlamm, der einen grossen Teil der am Ende des Prozesses vorliegenden Biomasse enthält, wird über eine Leitung 75 in einen Behälter 7b (Schlammmulde) ausgetragen. Mittels dieses Behälters 7b bzw. 7b' wird der Schlamm beispielsweise in eine nachfolgende, nicht dargestellte Kompostierstufe weitertransportiert. Der Transport des Behälters 7b, 7b' ist durch den Doppelpfeil 76 angedeutet.

Nach einer Retentionszeit in den Vergärungsstufen von rund zehn Tagen wird der "ausgefaulte Schlamm" in einen Speicherbehälter 209 transportiert. Dies geschieht wieder über den Transportkanal 20a und die zwangsfördernde Pumpe 20b.

Sollte der Geruch des entwässerten Schlamms noch störend sein, so ist der Behälter 209 als Belüftungsstufe vorzusehen. Dazu wird Luft aus einem Drucklufterzeuger 9 über eine Leitung 91 und Düsen 92 in den Schlamm eingeblasen. Unter Zufuhr der Luft - und bei Abschluss gegen die Umgebung - wird der Schlamm biologisch oxidiert. Die Abluft (Leitung 93) wird mittels nicht dargestellter Biofilter von Gerüchen befreit.

Im Behälter 209 kann der Schlamm durch Sedimentation eingedickt werden. Das dabei freigesetzte Wasser kann in den Wasserspeicher 7a eingeleitet werden (nicht dargestellt), womit es zumindest teilweise wieder in das Verfahren rückgeführt wird. Der eingedickte Schlamm kann je nach Beschaffenheit direkt zur Kompostierung gebracht oder zuvor mittels einer Presse 7 weiter entwässert werden.

Das in der Anlage 1 ablaufende Verfahren, das eine Hydrolysestufe und ein mehrstufiges Vergärungsverfahren umfasst, wird getaktet durchgeführt. Das zu behandelnde Material wird jeweils nach zeitlichen Intervallen von 1 bis 3 Tagen über den Transportkanal 20a, der die Reaktoren 200 bis 203 verbindet, und mittels der zwangsfördernden Pumpe 20b transportiert. Am Ende eines zeitlichen Intervalls wird in dem Schlammspeicher 209, der auch mit dem Transportkanal 20a in Verbindung steht, durch Entnahme von Schlamm Platz geschaffen. Sodann wird aus der letzten Vergärungsstufe (Reaktor 203) Schlamm in den Schlammspeicher transportiert und anschliessend wird Material sukzessive von Reaktor zu Reaktor verschoben. Nach der Materialentnahme aus der Hydrolysestufe wird in diese organisches Rohmaterial eingespeist, welches zuvor durch Versetzung mit Frischwasser oder Kreislaufwasser auf einen Feststoffanteil von rund 15 - 25% TS verdünnt worden ist.

Mit der Anlage 1 lässt sich das erfindungsgemässe Verfahren flexibel gestalten. Es kann von einem beliebigen Behälter 300 bis 303 sowie 309 in jeden anderen dieser Behälter Material verschoben werden. Da die Teilprozesse des Verfahrens von der Zusammensetzung des organischen Rohmaterials abhängen, kann es wichtig sein, gelegentlich von einer Routine-Verfahrensführung abzuweichen und beispielsweise von der zweiten oder dritten Vergärungsstufe Biomasse in eine vorangehende Stufe zurückzuspeisen. Bei einer Verarmung an Biomasse in einer Stufe kann diesem Mangel abgeholfen werden, indem man aus dem Speicher 309 Schlamm entnimmt und in die betreffende Stufe einspeist.

Man kann auch eine weit grössere Anzahl von Reaktorbehälter als in der beschriebenen Anlage 1 vorsehen. Es besteht dann die Möglichkeit, beispielsweise eine der Vergärungsstufen parallel in zwei Reaktoren ablaufen zu lassen. Auch kann nach Bedarf von einer Parallelschaltung einzelner Stufen der Verfahrensführung zu einer Serieschaltung und umgekehrt gewechselt werden.

Für ein optimales Verfahren müssen verschiedene Parameter der einzelnen Stufen eingestellt werden. Bei diesen Parametern handelt es sich insbesondere um pH-Werte, Temperaturen, Verweilzeiten und Konzentrationen. Die erfindungsgemässe Anlage ermöglicht es dank der besonderen Schaltung, die Parameter jeder Stufe unabhängig von den anderen Stufen zu steuern.

## Patentansprüche

1. Vergärungsanlage (1) zum Vergären von organischen Abfällen, eine Beschickungsvorrichtung (3), diverse Reaktoren (200, 201, 202, 203), mindestens einen Speicherbehälter (209), eine Entwässerungseinrichtung (7) und Fördermittel (45, 52, 210, 20a, 20b) als Anlagekomponenten umfassend, wobei die Beschickungsvorrichtung aus einer stationären Ladeeinrichtung (4) und einem an die Ladeeinrichtung ankoppelbaren Transportbehälter (30, 30') für die Abfälle besteht, die Reaktoren für eine Hydrolyse sowie auf diese folgende Vergärungsstufen vorgesehen sind, in der Entwässerungseinrichtung ein bei der Vergärung als Rest bleibender Bioschlamm eindickbar ist und die Fördermittel Verbindungen zwischen den Anlagekomponenten herstellen,
dadurch gekennzeichnet, dass jeder als Reaktor oder Speicher dienende Behälter über eine Förderleitung (210) mit einem gemeinsamen Transportkanal (20a) verbunden ist, wobei am Austrittsende des Transportkanals eine zwangsfördernde Pumpe (20b) angeordnet ist, mittels der zu behandelndes Material aus dem Transportkanal gezielt in jeden der genannten Behälter oder in die Entwässerungseinrichtung einspeisbar ist.

2. Anlage nach Anspruch 1, dadurch gekennzeichnet, dass der Entwässerungseinrichtung (7) ein Wasserspeicher (7a) und ein Wasserkreislauf (71, 74) zugeordnet sind, mittels dessen den einzelnen oder allen Reaktoren (200 bis 203) Wasser aus der Schlammeindickung (7) zuführbar ist.

3. Anlage nach Anspruch 1 oder 2, dadurch gekennzeichnet, dass die Reaktoren (200 bis 203) an ein Heizsystem (6, 62a, 60, 63, 260, 62b) angeschlossen sind, das einen Kreislauf mit einem Wärmeträger umfasst, der durch Verbrennen des gebildeten Biogases erhitzbar ist, wobei der Kreislauf für eine Wärmezufuhr in Wärmetauscher (260) der Reaktoren vorgesehen ist.

4. Anlage nach einem der Ansprüche 1 bis 3, dadurch gekennzeichnet, dass ein belüftbarer Speicherbehälter (209) vorgesehen ist, in dem die gebildete Biomasse oxidierbar ist.

5. Verfahren zum Behandeln eines organischen Rohmaterials in einer Anlage (1) gemäss einem der Ansprüche 1 bis 4, dadurch gekennzeichnet, dass die Behandlung zuerst in einer Hydrolysestufe in einem ersten Reaktor (200) und anschliessend in einem mehrstufigen Vergärungsverfahren in weiteren Reaktoren (201, 202, 203) durchgeführt wird, wobei das zu behandelnde Material getaktet nach zeitlichen Intervallen von 1 bis 3 Tagen über den die Reaktoren verbindenden Transportkanal (20a) und mittels der zwangsfördernden Pumpe (20b) transportiert wird.

6. Verfahren nach Anspruch 5, dadurch gekennzeichnet, dass am Ende eines zeitlichen Intervalls in einem Schlammspeicher (209), der mit dem Transportkanal (20a) in Verbindung steht, durch Entnahme von Schlamm Platz geschaffen wird, dass aus der letzten Vergärungsstufe Schlamm in den Schlammspeicher transportiert wird und dass anschliessend sukzessive von Reaktor zu Reaktor Material verschoben wird.

7. Verfahren nach Anspruch 6, dadurch gekennzeichnet, dass nach der Materialentnahme aus der Hydrolysestufe in diese organisches Rohmaterial eingespeist wird, welches zuvor durch Versetzung mit Frischwasser oder Kreislaufwasser auf einen Feststoffanteil von rund 15 - 25% TS verdünnt worden ist.

8. Verfahren nach einem der Ansprüche 5 bis 7, dadurch gekennzeichnet, dass beim Beschicken der Anlage (1) das zugeführte organische Rohmaterial in einem ersten Schritt zerkleinert und mit Wasser versetzt wird, welches aus dem Verfahren rückgeführt und bei einer Schlammentwässerung (7) freigesetzt worden ist.

9. Verfahren nach einem der Ansprüche 5 bis 8 und Anspruch 6, dadurch gekennzeichnet, dass in den Schlammspeicher (209) Luft zur Oxidierung der Biomasse eingeblasen wird.

10. Verfahren nach einem der Ansprüche 5 bis 9, dadurch gekennzeichnet, dass in den Verfahrensstufen freigesetztes Biogas zumindest teilweise als Wärmequelle für die im Verfahren benötigten Temperaturerhöhungen verwendet wird.
